# EUROPEAN PATENT SPECIFICATION

(11) **EP 3 271 730 B1**
(45) Date of publication and mention of the grant of the patent: **09.10.2019**
(21) Application number: 16722417.9
(22) Date of filing: 21.03.2016
(51) Int. Cl.: G01N 33/68, A61K 38/28, C12Q 1/6883

(54) **NOVEL MARKER FOR GESTATIONAL DIABETES**
NEUARTIGER MARKER FÜR SCHWANGERSCHAFTSDIABETES
NOUVEAU MARQUEUR DU DIABÈTE GESTATIONNEL

(30) Priority: 20.03.2015 NL 2014501
(43) Date of publication of application: 24.01.2018
(73) Proprietor: IQ Products B.V., 9727 DL Groningen (NL)
(72) Inventor: SCHUITEMAKER, Joost Henric Nicolaas, 9727 DL Groningen (NL); CREMERS, Thomas Ivo Franciscus Hubert, 9727 DL Groningen (NL)
(74) Representative: V.O.
(86) International application number: PCT/NL2016/050196
(87) International publication number: WO 2016/153344

(56) References cited:
- US-A1- 2013 274 123
- US-A1- 2014 273 024
- "Enzyme-linked Immunosorbent Assay Kit For Secreted Frizzled Related Protein 4 (SFRP4)", , 1 July 2013 (2013-07-01), pages 1-8, XP055208007, Retrieved from the Internet: URL:http://www.cloud-clone.us/manual/ELISA -Kit-for-Secreted-Frizzled-Related-Protein -4-(SFRP4)-E95878Hu.pdf [retrieved on 2015-08-17] cited in the application
- TAMAN MAHDI ET AL: "Secreted Frizzled-Related Protein 4 Reduces Insulin Secretion and Is Overexpressed in Type 2 Diabetes", CELL METABOLISM, vol. 16, no. 5, 1 November 2012 (2012-11-01), pages 625-633, XP055208002, ISSN: 1550-4131, DOI: 10.1016/j.cmet.2012.10.009 cited in the application
- MICHAEL M HOFFMANN ET AL: "Association of secreted frizzled-related protein 4 (SFRP4) with type 2 diabetes in patients with stable coronary artery disease", CARDIOVASCULAR DIABETOLOGY, BIOMED CENTRAL, LONDON, GB, vol. 13, no. 1, 19 November 2014 (2014-11-19), page 155, XP021204126, ISSN: 1475-2840, DOI: 10.1186/S12933-014-0155-2 cited in the application
- ZHENWEN ZHANG ET AL: "Endogenous galanin as a novel biomarker to predict gestational diabetes mellitus", PEPTIDES, vol. 54, 1 April 2014 (2014-04-01), pages 186-189, XP055208000, ISSN: 0196-9781, DOI: 10.1016/j.peptides.2014.01.024 cited in the application
- CHAN T-F ET AL: "Increased serum retinol-binding protein 4 concentrations in women with gestational diabetes mellitus", REPRODUCTIVE SCIENCES, SAGE PUBLICATIONS, INC, US, vol. 14, no. 2, 1 February 2007 (2007-02-01), pages 169-174, XP008114582, ISSN: 1933-7205, DOI: 10.1177/1933719106298407 cited in the application
- SEYMOUR JAMIE V DE ET AL: "Early pregnancy metabolite profiling discovers a potential biomarker for the subsequent development of gestational diabetes mellitus", ACTA DIABETOLOGICA, SPRINGER INTERNATIONAL, BERLIN, DE, vol. 51, no. 5, 27 July 2014 (2014-07-27), pages 887-890, XP035398864, ISSN: 0940-5429, DOI: 10.1007/S00592-014-0626-7 [retrieved on 2014-07-27]

## Description

### 1. FIELD OF THE INVENTION

The present invention relates to novel methods of typing a female subject as being at risk of developing gestational diabetes. In particular, the present invention relates to the use of expression levels of Secreted Frizzled-Related Protein 4 (sFRP4) for typing a subject.

### 2. BACKGROUND OF THE INVENTION

Diabetes mellitus comprises a group of complex metabolic diseases which are characterized by (chronic) hyperglycemia due to defects in insulin secretion, action or both. Several types of diabetes mellitus are known which share common symptoms but have different underlying causes, ranging from autoimmune destruction of the β-cells of the pancreas to genetic defects in insulin action. Consequently, this disease is classified as type 1 diabetes (T1D), type 2 diabetes (T2D), gestational diabetes mellitus (GDM) and other types of diabetes (Ashcroft and Rorsman. Cell 2012; 148:1160-1171; Taylor and Agius. Biochem J 1988; 250:625-640; Kelly et al., Mol Pathol 2003; 56:1-10).

GDM is defined as glucose intolerance that is first identified during pregnancy and in the majority of cases it resolves after delivery. However, GDM is a major health problem affecting about 1-10% of all pregnancies while its incidence is increasing further (Lapolla et al., Nutr Metab Cardiovasc Dis 2009; 19:674-682). Additionally, GDM is associated with serious complications for infants and mothers and results in a higher risk of T2D after pregnancy Gilmartin et al., Rev Obstet Gynecol 2008; 1:129-134; Coustan, Clin Chem 2013; 59:1310-1321; Reece et al., Lancet 2009; 373:1789-1797). Considering the increasing incidence of GDM, it's prediction early during pregnancy is of paramount importance. GDM is currently diagnosed through prenatal screening, rather than reported symptoms. To enable the early prediction of GDM, a facile screening method based on a robust biomarker is, therefore, required. Although several biomarkers for the prediction of T1D and T2D are known (Salomaa et al., PLoS One 2010; 5:e10100; Wang-Sattler et al., Mol Syst Biol 2012; 8:615; Rathcke and Vestergaard, Cardiovasc Diabetol 2009; 8:61; Sattar et al., Diabetes 2004; 53:2855-2860; Vozarova et al., Diabetes 2002; 51:1889-1895; Riaz et al., J Pharm Biomed Anal 2010; 51:1103-1107; Shin et al., Diabetes Care 2001; 24:733-737; Lee et al., Cell 2013; 153:413-425), only a few potential biomarkers for the prediction of GDM have been described (Chan et al., Reprod Sci 2007; 14:169-174; Zhang et al., Peptides 2014; 54:186-189). Hence, novel and blood-detectable GDM biomarkers are urgently needed. Early screening to determine the risk to develop GDM or any other pregnancy related problem will limit the potential risk of an adverse pregnancy outcome, might limit problems after pregnancy and allow better use of health care resources.

### 3. SUMMARY OF THE INVENTION

The invention therefore provides a method of typing a pregnant female subject as being at risk of developing gestational diabetes, the method comprising a) providing a biological sample from the subject; b) determining a level of expression of Secreted Frizzled-Related Protein 4 (sFRP4) in said sample; c) comparing the determined level of expression of sFRP4 with a level of expression of sFRP4 in a reference sample; and d) typing said subject as being at risk of developing gestational diabetes if the level of expression of sFRP4 is increased in the sample of the subject, when compared to the reference sample. Said reference sample preferably is obtained from a female subject who did not develop gestational diabetes during pregnancy.

With regard to this new biomarker, it is of interest to note that several recent studies have indicated secreted frizzled-related protein 4 (sFRP4) a promising candidate in the prognosis of T2D (Hoffmann et al., Cardiovasc Diabetol 2014; 13:155; Mahdi et al., Cell Metab 2012; 16:625-633; Taneera et al., Cell Metab 2012; 16:122-134; Ebert et al., Diabet Med 2014; 31:1014-1017). The precise cause of GDM is as yet unknown. However, accumulating evidence suggests that GDM is a completely different form of diabetes which is characterized by a mix of several types of pathologies and with more than one potential cause, ranging from a hormonal origin to an autoimmune component. (Huang et al., Endocrinology 2009; 150:1618-1626; Ramos-Roman, Horm Metab Res 2011; 43:593-600; Collares et al., Mol Biol Rep 2013; 40:5351-5358; Evangelista et al., BMC Med Genomics 2014; 7:28; Mahmoud et al., Am J Reprod Immunol 2005; 53:21-29; Oztekin, Med Hypotheses 2007; 69:526-530; Steinborn et al., Am J Reprod Immunol 2006; 56:124-134).

In contrast to diabetes types 1 and 2, which are incurable chronic conditions, GDM is a reversible condition and blood glucose levels return to normal levels in most cases within a few days after delivery.

Mahmoud et al. (Mahmoud et al., Am J Reprod Immunol 2005; 53:21-29) revealed that GDM has an autoimmune component. They showed an imbalanced immune system in pregnant women with GDM compared to that of healthy pregnancies. This is also confirmed on the transcriptome level (Collares et al., Mol Biol Rep 2013; 40:5351-5358; Evangelista et al., BMC Med Genomics 2014; 7:28)]. Özer Oztekin and co-workers correlated these changes in the immune system with the presence of Human leukocyte antigen-G (HLA-G) (Özer Oztekin, Medical Hypotheses 2007: 69:526-530). Conceivably, GDM may be triggered by an antigenic load which is the fetus itself. Interestingly, expression of HLA-G by the fetus confers protection against the maternal immune system by down-regulating the activity of cytotoxic T cells towards trophoblast antigens. It has been suggested that this impairment of maternal cytotoxic T cell activity may also protect the islet cells of the pancreatic tissue.

This scenario is supported by the findings of Steinborn et al. (Steinborn et al., Am J Reprod Immunol 2006; 56:124-134), which demonstrated an increase in HLA-II antibodies in the maternal circulation. GDM is also thought to be caused when insulin receptors do not function properly. This is likely due to pregnancy-related factors such as the presence of human placental lactogen that interferes with susceptible insulin receptors (Huang et al., Endocrinology. 2009; 150:1618-265). This in turn causes inappropriately elevated blood sugar levels. Placental lactogen is not present in the system in periods other than pregnancy (Ramos-Román, Horm Metab Res 2001; 43:593-600).

Other factors have been described in type two diabetes that have no value in GDM. Schaller et al. (Schaller et al., Eur J Clin Invest 2010; 40:339-343) have, for instance, published the for type two earlier described YKL-40 as having no involvement in the mechanisms of GDM. Also for liver transaminases, like Gamma-glutamyltransferase, alanine transaminase and aspartate transaminase, it was published that they do not predict GDM in contrast to type 2 diabetes (Tan et al., Clinical Biochemistry 2012; 45:1192-1196). Despite a link between GDM and T2D, an association between sFRP4 and GDM is therefore not obvious.

sFRP4 is a recently identified cytokine of about 40 kDa and belongs to the family secreted frizzled-related proteins (Bergmann and Sypniewska, Clin Biochem 2014; 47:529-532). These secreted frizzled-related proteins act as modulators of the Wnt signalling pathway. In addition to its role as Wnt antagonist, sFRP4 has been implicated in the inhibition of angiogenesis, tumor suppression and pre-eclampsia (Bergmann and Sypniewska, Clin Biochem 2014; 47:529-532). Moreover, this protein was shown to play a role in the regulation of glucose resistance by controlling the insulin secretion from islet cells (Mahdi et al., Cell Metab 2012; 16:625-633).

It is now shown that an increased sFRP4 concentration is present in the serum of pregnant women that will develop GDM, compared to serum samples of healthy controls, women that will not develop GDM or other pregnancy related problem. These data show that this protein is of potential clinical value as biomarker for the prediction of GDM during early pregnancy.

The biological sample preferably is a bodily fluid, preferably blood, including plasma and/or serum.

The biological sample is preferably obtained before 20 weeks of gestation, more preferred before 18 weeks of gestation, more preferred before 16 weeks of gestation, more preferred before 14 weeks of gestation, more preferred before 12 weeks of gestation. Said biological sample is preferably obtained from a pregnant mammal during the first half of gestation, preferably during the first one third period of gestation. In human, said biological sample is preferably obtained from a pregnant female during the first trimester of gestation.

A level of expression of sFRP4 is preferably determined by a biosensor, preferably by using a disposable cartridge. A preferred level of expression is determined of sFRP4 protein, preferably by Enzyme-Linked Immuno Sorbent Assay (ELISA).

A dietary meal plan and/or physical activity for use in a method of treatment of a subject that has been typed as being at risk of developing gestational diabetes according to the methods of the invention.

Metformin, a sulfonylurea, a thiazolidinedione, a DPP-4 inhibitor, a GLP-1 receptor agonist, and/or a SGLT2 inhibitor, for use in a method of treatment of a subject that has been typed as being at risk of developing gestational diabetes according to the methods of the invention.

Insulin for use in a method of treatment of a subject that has been typed as being at risk of developing gestational diabetes according to the methods of the invention.

The invention further provides the use of a kit for assessing a pregnant female subject for susceptibility to gestational diabetes, said kit comprising a device for collecting a test sample from a patient and reagents for measuring the expression of Secreted Frizzled-Related Protein 4 (sFRP4) in said sample, wherein the reagents are for real-time PCR or an immunochemical assay.

Said reagents for measuring expression of sFRP4 preferably include a receptacle that is coated with an antibody specific to sFRP4, preferably a microtiter plate that is coated with an antibody specific to sFRP4. Said receptacle that is coated with an antibody specific to sFRP4 is preferably used in an Enzyme-Linked Immuno Sorbent Assay (ELISA).

The invention further provides the use of a kit according to the invention, for typing a female subject as being at risk of developing gestational diabetes.

The invention further provides the use of an antibody specific to sFRP4 in an in vitro method for determining a risk of a female subject of developing gestational diabetes.

### 4. LEGEND TO FIGURES

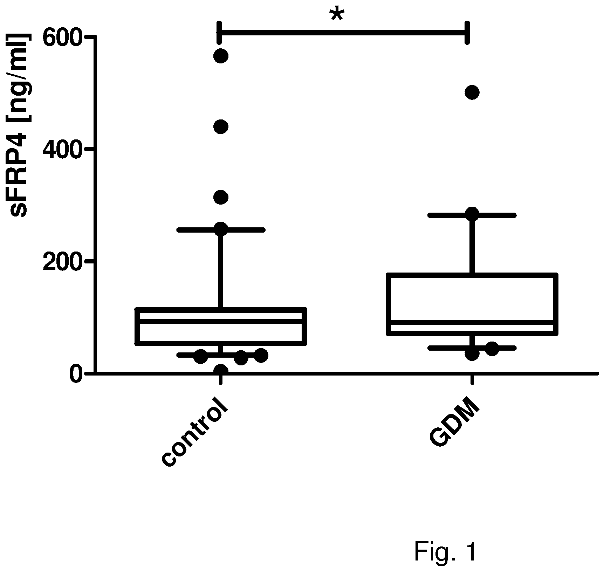

Figure 1: sFRP4 concentration at gestation age 80 days (95% CI 79 - 82 days). *= p<0.05.

### 5. DETAILED DESCRIPTION OF THE INVENTION

### 5.1 Definitions

The term "gestational diabetes", or gestational diabetes mellitus, (GDM), as are used herein, refers to a condition in which a woman without previously diagnosed diabetes exhibits high blood glucose levels during pregnancy, especially during their third trimester. A subject suffering from gestational diabetes has serum or plasma glucose values above 130 mg per dL (7.2 mmol per L), or even above 140 mg per dL (7.8 mmol per L).

The term "female subject", as are used herein, refers to a mammalian female, preferably a human female.

The term "biological sample", as is used herein, refers to a bodily fluid or a tissue sample such as, for example, hair, skin, or a biopsy sample, from a pregnant female subject. Said bodily fluid preferably is selected from cerebrospinal fluid, saliva, sweat, sputum, lymph, urine, sputum, vaginal secretions, perspiration, buccal swabs, and blood, including blood plasma and blood serum. Plasma is prepared by removing red and white blood cells from blood, for example by centrifugation, whereby coagulation of blood is prevented by using, for instance, heparin, citrate or EDTA. Serum is prepared by formation of a blood clot, and removal of the clot using, for example, a centrifuge. A preferred biological sample is or comprises serum.

The term "Secreted Frizzled-Related Protein 4" or sFRP4, as is used herein, refers to a gene that encodes of about 346 amino acids with a predicted molecular weight of 39.9 kDa and an actual molecular weight of approximately 50-55 kDa. sFRP4 belongs to a family of five sFRPs that are characterized by a secretion signal peptide at the N-terminus, which is followed by a ∼120 amino acid cysteine-rich domain (CRD). Expression sFRP4 can be detected in various normal tissues including endometrium, ovary, kidney, heart, brain, mammary gland, cervix, pancreas, stomach, colon, lung, skeletal muscle, testis, eye, bone, prostate, and liver. Human sFRP4 is represented by UniProt accession number Q6FHJ7, which is encoded by the RefSeq transcript accession number NM_003014.

The term "biosensor", or "biological sensor", as is used herein, refers to a sensing device in which a biologically derived signal is coupled to a transducer, allowing quantification of the biologically derived signal. The signal that is generated when the sensor interacts with an analyte, in this case an expression product of sFRP4, preferably sFRP4 protein, may be electrical, optical or thermal. It is converted by means of a suitable transducer into a measurable electrical parameter such as a current or voltage.

The term "expression product of sFRP4", as is used herein, refers to a RNA or protein expression product of sFRP4. The terms "expression product", "RNA" and "protein" refer to the full length products as well as breakdown expression products such as breakdown RNA and protein products.

The term "Enzyme-linked immunosorbent assay (ELISA)", as is used herein, refers to a plate-based assay that is designed for detecting and quantifying antigens such as sFRP4. In a competition ELISA, known amounts of an antigen are immobilized to a surface. A sample comprising unknown amounts of sFRP4 is added, and sFRP4 is subsequently complexed with an antibody that is preferably conjugated, directly or indirectly, to a detectable label such as a colorimetric label, a fluorescent label, a radioactive label or a chemiluminescent label, or an enzyme. Following washing, detection of antibody that is complexed to the immobilized sFRP4 is accomplished by assessing the conjugated label or enzyme activity via incubation with a substrate to produce a measureable product. The amount of label or enzyme activity is inversely proportional to the amount of sFRP4 in the sample. A preferred assay is a sandwich ELISA, in which a receptacle is coated with an antibody specific to sFRP4, termed "capture antibody", and detection of bound sFRP4 is accomplished with a second antibody, termed "detection antibody". It is preferred that the capture and detection antibodies do not interfere with each other and can bind simultaneously to sFRP4. Said second antibody is preferably directly or indirectly conjugated to a detectable label such as a colorimetric label, a fluorescent label a radioactive label, or a chemiluminescent label, or an enzyme. Detection of the amount of enzyme-conjugated antibody is preferably performed by incubation with a substrate to produce a measureable product. As an alternative, turbidimetric assays are preferred, especially for competition ELISAs.

The term "directly conjugated with a detectable label", as used herein, refers to the labeling of the antibody itself with a detectable label.

The term "indirectly conjugated with a detectable label", as used herein, refers to the indirect labeling of an antibody, for example using a biotin-labelled antibody and a detectable label that is bound to streptavidin, or by using a further antibody that is directed against the indirectly labeled antibody and which further antibody is labeled with a detectable label.

The term "antibody", as used herein, refers to a polyclonal or monoclonal whole immunoglobulin, e.g., IgG, IgM, IgA, IgE and the like, or an immunoglobulin fragment, e.g., isolated CDR regions; single chain Fv molecules ("scFv"), wherein a VH domain and a VL domain are linked by a peptide linker that allows the two domains to associate to form a binding domain, diabody (Hollinger et al., 1993. PNAS USA 90: 6444-6448), F(ab)2, F(ab')2, Fab, Fab' and the like, or a mixture thereof. Antibodies and antibody fragments which specifically bind a wide variety of ligands are known, and many of these are disclosed in the patents. In addition, the term "antibody" includes artificial antibody-like molecules or antibody mimetics such as APTAMERS (Que-Gewirth and Sullenger, 2007. Gene Therapy 14, 283-291); AFFIBODY® molecules (Nord et al., 1995. Prot Eng 8: 601-608), ANTICALINS® (Skerra, 2008. FEBS J. 275: 2677-2683), and AVIMERS® (Silverman et al., 2005. Nat Biotechnol 23: 1556-1561). The term antibody also includes other proteinaceous molecules that bind sFRP4 including, for example, Wingless 7a or a sFRP4-binding part thereof.

The term "dietary meal plan", as is used herein, as is used herein, refers to a diet that is rich in fiber, with a variety of fruit and vegetables, and low in both sugar and fat, especially saturated fat.

The term "physical activity", as is used herein, refers to light or moderate physical activity, including walking, hiking, climbing stairs, swimming or taking a water-aerobics class, dancing, and riding a bicycle outdoors or a stationary bicycle indoors, that helps to lower blood glucose levels. Especially muscle activity may help lowering and/or preventing high blood glucose.

The term "reference sample", as used herein, preferably refers to sample from a female subject that did not develop gestational diabetes during pregnancy. The skilled person will understand that the term reference sample may also refer to the level of expression of sFRP4 that is determined in a female subject that did not develop gestational diabetes during pregnancy, or the average level of expression of sFRP4 that is determined in a group of female subjects that did not develop gestational diabetes during pregnancy. It is preferred that said group of female subject includes more than 10 subjects, more preferred more than 20 subjects, more preferred more than 30 subjects. These subjects preferably have the same physical characteristics, for example age, weight, gestational age and BMI as the female subject of which the level of expression of sFRP4 is to be determined. In one embodiment, said reference sample refers to a serum or plasma sFRP4 concentration of 130 ± 12 ng/ml standard error of the mean (SEM), as determined with an ELISA kit for Secreted Frizzled Related Protein 4 (SFRP4) of Cloud-Clone Corp., Houston, TX).

### 5.2 Methods of diagnosis

The present invention is directed to sFRP4 for use as a biomarker for the prediction of GDM during early pregnancy. It is shown that an increased sFRP4 concentration in serum of healthy, pregnant women that show no sign of diabetes, as compared to the sFRP4 concentration in serum of matched women that are of the same age, gestational age, weight and BMI, is indicative for women that will develop GDM later during pregnancy. Early detection of women at risk of developing GDM, and timely therapeutic intervention, are crucial for improved outcome of patients that are likely to suffer from GDM. The early detection will allow the early start of a specific therapy that is targeted to normalizing glucose levels in those women.

Recently, the SFRP4 gene was identified in a systems genetic study as one of the genes associated with pancreatic islet dysfunction (Taneera et al., 2012. Cell Metab 16: 122-134). Furthermore, it was reported that sFRP4 is overexpressed in pancreatic islet cells of TD2 patients, while its increased circulatory concentrations impair insulin secretion by β cells and thereby reduce glucose tolerance (Mahdi et al., 2012. Cell Metab 16: 625-633). This points towards a direct role of sFRP4 in controlling the insulin secretion from islet cells. In addition to its effect on glucose metabolism, elevated levels of sFRP4 also impair triglyceride metabolism in patients with TD2 (Hoffmann et al., 2014. Cardiovasc Diabetol 13: 155). Despite a clear link between sFRP4 and T2D, an association between sFRP4 and GDM has not been reported before and is not immediately obvious because the cause of GDM is as yet unclear. In contrast to the often suggested link between GDM and T2D, a hormonal origin as well as an autoimmune component have been proposed (Huang et al., 2009. Endocrinology 150: 1618-1626; Ramos-Roman, 2011. Horm Metab Res 43: 593-600; Collares et al., 2013. Mol Biol Rep 40: 5351-5358; Evangelista et al., 2014. BMC Med Genomics 7: 28; Mahmoud et al., 2005. Am J Reprod Immunol 53: 21-29; Oztekin, 2007. Med Hypotheses 69: 526-530; Steinborn et al., 2006. Am J Reprod Immunol 56: 124-134). This suggests that that GDM is a completely different form of diabetes with more than one potential cause and a mix of several types of pathologies, which are all diagnosed as GDM.

sFRP4 for use as a biomarker for the prediction of GDM during early pregnancy is independent of BMI (see Table 3). However, it was found that a higher BMI, such as above 25 (overweight) or above 30 (obese), increases the predictive value of the marker. Therefore, the pregnant female subject preferably has a BMI above 20, preferably above 25, preferably above 30.

Methods to obtain a biological sample, preferably a bodily fluid, from a female subject, preferably a human female, are known in the art. Preferred methods include the provision of a blood sample, preferable serum.

It is further preferred that the sample comprises RNA expression products and/or protein expression products. Such biological sample can be obtained in numerous ways, as is known to a skilled person. For example, the biological sample can be freshly prepared from a bodily fluid, preferably serum, at the moment of harvesting, or it can be prepared from samples that are stored at -70°C until processed for sample preparation. Alternatively, said bodily fluid can be stored under conditions that preserve the quality of the protein or RNA. Examples of these preservative conditions are fixation using e.g. formaline and paraffin embedding, RNase inhibitors such as RNAsin® (Pharmingen) or RNasecure® (Ambion), aquous solutions such as RNAlater® (Assuragen; US06204375), Hepes-Glutamic acid buffer mediated Organic solvent Protection Effect (HOPE; DE 10021390), and RCL2 (Alphelys; WO04083369), and non-aquous solutions such as Universal Molecular Fixative (Sakura Finetek USA Inc.; US7138226).

It is preferred that the level of expression of sFRP4 is normalized. Normalization refers to a method for adjusting or correcting a systematic bias in the measurements. Systemic bias results in variation by differences in overall performance, for example between ELISA plates, which can be due to, for example, inconsistencies in fabrication, immobilization, and variation between samples, which can be due, for example, to variations in purity. Systemic bias can also be introduced during the handling of a sample.

Conventional methods for normalization of expression data rely on the detection of expression products of genes such as glyceraldehyde-3-phosphate dehydrogenase, transferrin and albumin, of which the expression level is thought to be constant in a given sample and independent from the developmental stage or history of said sample.

RNA may be isolated from a biological sample by any technique known in the art, including but not limited to Trizol (Invitrogen; Carlsbad, California), RNAqueous® (Applied Biosystems/Ambion, Austin, Tx), Qiazol® (Qiagen, Hilden, Germany), Agilent Total RNA Isolation Lits (Agilent; Santa Clara, California), RNA-Bee® (Tel-Test. Friendswood, Texas), and Maxwell™ 16 Total RNA Purification Kit (Promega; Madison, Wisconsin). A preferred RNA isolation procedure involves the use of Qiazol® (Qiagen, Hilden, Germany). RNA can be extracted from a whole sample or from a portion of a sample generated by, for example section or laser dissection.

The level of RNA expression of Secreted Frizzled-Related Protein 4 (sFRP4) can be determined by any method known in the art. Methods to determine RNA levels of genes are known to a skilled person and include, but are not limited to, Northern blotting, quantitative Polymerase chain reaction (qPCR), also termed real time PCR (rtPCR), microarray analysis and RNA sequencing. The term qPCR refers to a method that allows amplification of relatively short (usually 100 to 1000 basepairs) of DNA sequences. For this, mRNA is converted into complementary DNA (cDNA) using a reverse transcriptase. cDNA is subsequently amplified by PCR with the aid of one or more primers that specifically hybridize to the sFRP4 cDNA. The amount of product that is amplified can be quantified using, for example, TaqMan® (Applied Biosystems, Foster City, CA, USA), Molecular Beacons, Scorpions® and SYBR® Green (Molecular Probes). Quantitative Nucleic acid sequence based amplification (qNASBA) can be used as an alternative for qPCR.

Alternative methods for the quantification of RNA expression levels include microarray analysis and next generation sequencing. For microarray analysis, a hybridization mixture is prepared by extracting and labelling of RNA. The extracted RNA is preferably converted into a labelled sample comprising either complementary DNA (cDNA) or cRNA using a reverse-transcriptase enzyme and labelled nucleotides. A preferred labelling introduces fluorescently-labelled nucleotides such as, but not limited to, cyanine-3-CTP or cyanine-5-CTP. Examples of labelling methods are known in the art and include Low RNA Input Fluorescent Labelling Kit (Agilent Technologies), MessageAmp Kit (Ambion) and Microarray Labelling Kit (Stratagene).

The methods of the invention for determining a level of RNA expression of sFRP4 preferably comprise the provision of a probe or a labeled primer that specifically hybridizes to a target nucleic acid region that is present on the sFRP4 mRNA or on cDNA or cRNA that is derived from the sFRP4 mRNA. Said probe or labeled primer preferably specifically hybridizes to an amplified product that is generated using first and second primers that specifically hybridize to a region on the sFRP4 mRNA or on cDNA or cRNA that is derived from the sFRP4 mRNA. The methods further comprise detecting a hybridization product comprising the probe or labeled primer and the sFRP4 mRNA or a relevant part thereof, or on cDNA or cRNA that is derived from the sFRP4 mRNA.

The term "specifically hybridizing", as is used herein, refers to a primer and/or a probe that is capable of hybridizing specifically under stringent hybridization conditions to a target nucleic acid template that is obtained or derived from sFRP4 mRNA. The length of the primer or probe is preferably more than 15 bases, but less than 100 bases, preferably between 16 and 30 bases. In general, a primer or probe is able to hybridize to a target nucleic acid template when the percentage of sequence identity of the molecule is at least 90% over substantially the whole length, more preferred at least 91%, more preferred at least 92%, more preferred at least 93%, more preferred at least 94%, more preferred at least 95%, more preferred at least 96%, more preferred at least 97%, more preferred at least 98%, more preferred at least 99%, more preferred 100% identical to a nucleic acid that is obtained or derived from said target nucleic acid template over substantially the whole length of the primer or probe. The term "substantially the whole length" is used to indicate that the probe may comprise additional nucleotide sequences, for example at the 5' and/or 3' ends that are not present in the gene or region described herein above.

The first and second primers preferably act together to amplify a region on the sFRP4 mRNA or on cDNA or cRNA that is derived from the sFRP4 mRNA. Said first and second primers are preferably provided in a reaction chamber. Said reaction chamber preferably further comprises a buffer, dNTPs, and/or DNA polymerase, preferably in a lyophilized form. Said level of RNA expression of sFRP4 is preferably determined by multiplex assay techniques, including multiplex PCR and bead-based multiplexing, allowing simultaneous detection of expression levels of sFRP4 and of, for example, normalization genes such as transferrin and albumin.

A preferred sample comprises protein expression products of sFRP4. Methods and means for extraction of proteins from a variety of samples are known in the art, including but not limited to lysis of tissue and/or cells with a mild detergent such as deoxycholic acid, and or a chaotropic agens such as sodium iodide and urea.

As a secreted protein, the level of expression of sFRP4 in a bodily fluid such as blood, including blood plasma and blood serum, is preferably determined directly in said sample, without isolation and/or extraction of proteins.

A level of expression of sFRP4 may be determined by polyacrylamide gel electrophoresis, including two dimensional gel electrophoresis, multidimensional protein identification technology, ELISA, bead-based immunoassays, immuno-PCR using, for example, Thunder-Link® antibody-oligonucleotide conjugation kit (Innova Biosciences. Cambridge UK), surface plasmon resonance, liquid chromatography-mass spectrometry (LC-MS), matrix-assisted laser desorption/ionization time-of-flight mass spectrometry (MALDI-TOF).

A level of expression of sFRP4 is preferably determined using a monoclonal and/or polyclonal antibody that is directed against sFRP4, preferably employing an enzyme-linked immunosorbent assay (ELISA). A preferred ELISA is a competition ELISA or a sandwich ELISA. A most preferred ELISA is a sandwich ELISA, in which the antigen (sFRP4) is immobilized to a surface by means of a capture antibody that specifically binds to sFRP4, and a detection antibody that is conjugated to an enzyme is used for detection of immobilized sFRP4. Said capture antibody and detection antibody preferably recognize and bind to distinct epitopes on sFRP4. Preferred ELISA kits include ELISA Kit for Secreted Frizzled Related Protein 4 (sFRP4) (Cloud-Clone Corp., Houston, USA), Human secreted frizzled-related protein 4, sFRP4 ELISA Kit (MyBioSource, Inc., San Diego, USA), and Human sFRP4 Elisa Kit, United States Biological, Swampscott, USA).

The level of expression of sFRP4 is preferably determined by a biosensor, preferably by using a disposable cartridge. A preferred biosensor provides methods and means for detecting a level of expression of sFRP4, preferably including antibodies that specifically bind to sFRP4. A preferred biosensor comprises a reusable hand-held reader capable of simple push-button operation for automated analysis of samples, and cost-effective disposable cartridges, preferably disposable microfluidic sensor cartridges, that have been functionalized to provide optimal quantification of multiple clinically relevant agents, including sFRP4. A preferred biosensor is a lateral flow test device, preferably a lateral flow device that allows to determine the quantity of sFRP4 in a sample, for example based on the accumulation of quantifiable substances such as magnetic particles. Said biosensor preferable allows point-of-care (POC) diagnosis.

Said level of expression of sFRP4 in a biological sample of a female subject is preferably compared to the level of expression of sFRP4 in a control, where the level of expression in said control preferably represents the average level of expression as determined in female subjects that did not suffer from gestational diabetes during pregnancy. An increase in the level of expression of sFRP4 in the sample from the female subject indicates that the subject is at risk of developing gestational diabetes in the second or third trimester of pregnancy. Those of skill in the art will readily see that said control level may alternatively represent the level of expression found in female subjects that did suffer from gestational diabetes during pregnancy. In that case, a decrease in the level of expression of sFRP4 in the sample from the female subject indicates that the subject has a reduced risk of developing gestational diabetes in the second or third trimester of pregnancy.

As an alternative, the concentration of sFRP4 may be determined in the sample of the subject, preferably in a serum sample of the subject. A serum concentration of sFRP4 above 130 pg/ml at the end of the first trimester, as determined with the ELISA Kit for Secreted Frizzled Related Protein 4 (sFRP4) (Cloud-Clone Corp., Houston, USA), is associated with an almost fourfold (OR 3.75) higher risk to develop GDM later in pregnancy, when compared to a subject having a serum concentration of sFRP4 below 130 pg/ml at the end of the third trimester.

The invention further provides a method of typing a female subject as being at risk of developing gestational diabetes, comprising
i) obtaining a biological sample from the subject;
ii) applying an antibody specific for Secreted Frizzled-Related Protein 4 (sFRP4) to the sample, wherein presence of sFRP4 creates an antibody-sFRP4 complex;
iii) applying a detection agent that detects the antibody-sFRP4 complex;
iv) determining the amount of detected antibody-sFRP4 complex and comparing said determined amount to the amount of antibody-sFRP4 complex that is present in a reference sample; and
v) typing said subject as being at risk of developing gestational diabetes, if the level of expression of sFRP4 is increased in the sample of the subject, when compared to the reference sample.

The invention further provides a method comprising i) obtaining a biological sample from a pregnant female subject comprising; ii) applying an antibody specific for Secreted Frizzled-Related Protein 4 (sFRP4) to the sample, wherein presence of sFRP4 creates an antibody- sFRP4 complex; iii) applying a detection agent that detects the antibody-sFRP4 complex; iv) determining the amount of detected antibody-sFRP4 complex. The invention further provides a kit for assessing a pregnant female subject for susceptibility to gestational diabetes, said kit comprising a device for collecting a biological sample from a patient and reagents for measuring a level of expression of Secreted Frizzled-Related Protein 4 (sFRP4) in said sample.

Said device for collecting a biological sample from a pregnant female subject preferably comprises a receptacle for collecting a bodily fluid from the patient, preferably for collecting blood from the subject and, if required, a needle for transferring blood directly from a vein to the receptacle. Said receptacle preferably is a tube, such as a serum separator tube. Said receptacle preferably comprises particles such as particles of amorphous porous glass material, or micronised silica particles, for enhancing clot activation of blood samples. The blood sample may then be centrifuged, allowing the clear serum to be removed for testing. Said particles may include platelet-like particles that are attached to human antibody fragments that recognize and bind to fibrin.

The reagents for determining a level of expression of sFRP4 in a kit according to the invention are preferably reagents for real-time PCR or reagents for an immunochemical assay.

Said reagents for real-time PCR preferably include reagents for extraction of nucleic acid, preferably RNA, from the biological sample, an/or reagents for rtPCR, including primers and probe for reverse transcription, amplification and detection of a part of the mRNA expression product of sFRP4.

The reagents for determining a level of expression of sFRP4 preferably include a receptacle, preferably a microtiter plate or a disposable cartridge, that is coated with a first antibody specific to sFRP4. A preferred receptacle is a microtiter plate, including a 6 well microtiter plate, a 12 well microtiter plate, a 24 well microtiter plate, a 48 well microtiter plate, 96 well microtiter plate, and a 284 well microtiter plate. Said reagents preferably further include a second antibody that binds to sFRP4. Said second antibody preferably is labeled with a detectable label. The term "detectable label", as is used herein, refers to a label that is detectable by spectroscopic, photochemical, biochemical, immunochemical, or chemical means. For example, useful labels include radioactive labels, fluorescent labels, electron-dense reagents, enzymes (as commonly used in ELISAs), biotin, or haptens and proteins for which antisera or monoclonal antibodies are available.

Said second antibody preferably does not or not substantially compete with the first antibody for binding to sFRP4. In that case, the amount of detectable label that is detected on the receptacle, if needed after repeated washing steps as is known to a skilled person, is used as a measure for the level of expression of sFRP4 in the biological fluid of the subject. If required, known amounts of sFRP4 protein, or of a part of the protein that is reactive with the first and second antibodies, can be used for providing a calibration curve, whereby the amounts of detectable label that is detected on the known amounts of sFRP4 protein is used for determining a level of expression of sFRP4 in the biological fluid of the subject. Said calibration curve may be determined in parallel, or in separate reactions.

The invention further provides an use of a kit according to the invention, for typing a female subject as being at risk of developing gestational diabetes.

The invention further provides an antibody specific to sFRP4 for use in a method for determining a risk of a female subject of developing gestational diabetes.

### 5.3 Methods of treatment

The invention further provides a dietary meal plan and/ or physical activity for use in a method of treatment of a subject that has been typed as being at risk of developing gestational diabetes according to the methods of the invention.

The term "treatment", as used herein, is aimed at reducing the level of expression and/or the activity of sFRP4, and/or at establishing preprandial blood glucose levels of between 70 and 130 mg/dl, and/or 2-hour postprandial blood glucose levels of below 180 mg/dl.

A subject of which the level of expression of sFRP4 is not increased when compared to a reference, and/or of which the serum or plasma concentration of sFRP4 is below 130 ± 12 ng/ml standard error of the mean (SEM), as determined with an ELISA kit for Secreted Frizzled Related Protein 4 (SFRP4) of Cloud-Clone Corp., Houston, TX), at the 20 weeks of gestation does not have an increased risk of developing gestational diabetes and may need no further treatment.

First-line therapy for women with gestational diabetes, or who are at risk of developing gestational diabetes, is dietary modification, often referred to as medical nutritional therapy. This is best done in consultation with an experienced nutritionist, and should take cultural preferences into account. Most programs involve carbohydrate counting, with meal- and snack-specific recommendations. Modifications in nutritional therapy are made based on patient preferences, amount (or lack) of weight gain, and glucose monitoring. Moderate exercise also may help in the management of gestational diabetes. Although medical nutritional therapy and exercise are safe, practical, and inexpensive interventions, their impact on patient outcomes has not been conclusively demonstrated in large RCTs.

Pharmacotherapy is indicated when medical nutritional therapy results in inadequate glucose control, lack of expected weight gain (as a result of calorie restriction), or when patients are consistently hungry. Pharmacotherapy is also indicated in the setting of elevated fasting glucose levels, because dietary modification has little effect on these levels.

Second-line therapy for women with gestational diabetes, or who are at risk of developing gestational diabetes, comprises treating a subject at risk of developing gestational diabetes with a sFRP4 antagonist, metformin, a sulfonylurea, a thiazolidinedione, a GLP-1 receptor agonist, and/or a SGLT2 inhibitor.

The term sFRP4 antagonist, as is used herein, refers to a molecule that inhibits the activity of sFRP4 including, but not limited to, Wingless 7a and, preferably, a sFRP4-binding part thereof, and/or an antibody that is directed against sFRP4. Further antagonists include cyclothiazide (6-Chloro-3,4-dihydro-3-(2-norbornen-5-yl)-2H-1,2-4-benzothiadiazine-7-sulfonamide 1,1-dioxide; Sigma-Aldrich), clopamide (4-chloro-N-(2,6-dimethylpiperidino)-3-sulphamoylbenzamide; Abcam) and perindopril ((2S,3AS,7as)-1-[(2S)-2-{[(2S)-1-ethoxy-1-oxopentan-2-yl]amino}propanoyl]octahydro-1H-indole-2-carboxylic acid; Sigma-Aldrich) (Bukhari et al., 2014. Molecules 19, 10129-10136). Cyclothiazide is preferably provided at a dosage of between 5 mg and 50 mg/day, preferably at 10-20 mg/day. Perindopril is preferably provided at a dosage of between 1 mg and 20 mg/day, preferably at 4-8 mg/day.

The term "metformin", as is used herein, refers to N,N-dimethylimidodicarbonimidic diamide, which is an oral antidiabetic drug in the biguanide class. It is gaining increasing acceptance as an alternative to insulin in the management of gestational diabetes. Metformin works by suppressing glucose production by the liver. Metformin is preferably provided at a dosage of between 100 mg and 3000 mg/day, preferably at 1000-2000 mg/day.

The term "sulfonylurea", as is used herein, refers to a class of hypoglycemic agents that increase the secretion of insulin by the pancreas. Examples of sulfonylureas are chlorpropamide, tolbutamide, glipizide, glyburide and glimepiride. Sulfonylurea is preferably provided at a dosage of between 1 mg and 20 mg/day, preferably at 5-10 mg/day.

The term "thiazolidinedione", or "glitazone", as is used herein, refers to a class of hypoglycemic agents that act as agonists of the peroxisome proliferator-activated receptors-gamma (PPARgamma), thereby altering the expression levels of several genes involved in glucose and lipid metabolism and energy balance. Examples of a thiazolidinedione include rosiglitazone, pioglitazone, lobeglitazone, troglitazone, netoglitazone, rivoglitazone, and ciglitazone. Thiazolidinedione is preferably provided at a dosage of between 1 mg and 50 mg/day, preferably at 15-30 mg/day.

The term "glucagon-like peptide-1 receptor agonist" or "GLP-1 receptor agonist", as is used herein, refers to a class of hypoglycemic agents that stimulate the release of insulin and reduce levels of glucagon. Examples of a GLP-1 receptor agonist include exenatide, liraglutide, lixisenatide, albiglutide, and taspoglutide. A GLP-1 receptor agonist is preferably provided at a dosage of between 1 microg and 50 microg/day, preferably at 5-10 microg/day.

The term "SGLT2 inhibitor" or "gliflozin", as is used herein, refers to a class of hypoglycemic agents that block the reabsorption of filtered glucose in kidneys, leading to the excretion of glucose in urine, thereby reducing blood glucose levels. Examples of a GLP-1 receptor agonist include dapagliflozin, canagliflozin, ipragliflozin, tofogliflozin, empagliflozin, remogliflozin etabonate, and ertugliflozin. Gliflozin is preferably provided at a dosage of between 1 mg and 500 mg/day, preferably at 5-100 mg/day.

Third-line therapy for women with gestational diabetes comprises treating a subject at risk of developing gestational diabetes comprises insulin therapy for women whose fasting glucose level exceeds 95 mg per dL, whose one-hour postprandial glucose level exceeds 130 to 140 mg per dL, or whose two-hour postprandial glucose level exceeds 120 mg per dL (6.65 mmol per L). Insulin is the first-line pharmacologic therapy for gestational diabetes. Most insulin regimens include intermediate-acting insulins, such as isophane (NPH), and short-acting insulins, such as regular recombinant (Humulin R) and the insulin analogues aspart (Novolog) and lispro (Humalog).

The term "insulin therapy", as is used herein, refers to the regular injection, i.e. daily or multiple times per day, of insulin and/or variants of insulin. Variants of insulin include rapid-acting insulin, such as aspart insulin, lispro insulin and glulisine insulin, which begin to work about 15 minutes after injection; short acting insulin, such as neutral insulin, which peaks in about 1 hour; intermediate-acting insulin, such as isophane, which start to lower blood glucose within 1 to 2 hours after injection; long acting insulin, such as insulin detemir and insulin glargine, which start to lower blood glucose within 4 to 6 hours after injection and has its strongest effect 10 to 18 hours after injection. Premixed insulin, comprising a mixture of an intermediate- or long-acting insulin, with a rapid-acting insulin, are often used for patients who cannot or who are not willing to use basal-bolus insulin, or for patients who are non-compliant with self-monitoring and titration of multiple insulin doses.

For the purpose of clarity and a concise description, features are described herein as part of the same or separate aspects and preferred embodiments thereof, however, it will be appreciated that the scope of the invention may include embodiments having combinations of all or some of the features described.

The invention will now be illustrated by the following example, which is provided by way of illustration and not of limitation and it will be understood that many variations in the methods described and the amounts indicated can be made without departing from the scope of the appended claims.

### Examples

### Example 1

### Materials and methods

### Subjects

In order to establish the value of maternal blood levels of sFRP4 as prognostic marker for the development of GDM samples were selected from a study database. This database contains samples drawn from the antecubital vein in serum collection tubes by phlebotomy during the first trimester of pregnancy. From the database, serum samples of 50 pregnant women who developed GDM during their pregnancy and 100 women with normal pregnancies were selected. The baseline characteristics of both study groups are displayed in Table 1, revealing no significant differences in gestational age, weight and BMI at the time of sampling. Both study groups are, therefore, well comparable in all parameters.

**Table 1. Patient characteristics of uncomplicated pregnancies and pregnancies affected by gestational diabetes (GDM)..**

| **Variable** | | **Uncomplicated pregnancies (n=96)** | **Gestational diabetes (n=49)** |
|---|---|---|---|
| Maternal age (years) | mean (SD) | 34.3 (5.0) | 34.3 (5.6) |
| Maternal weight (kg) | mean (SD) | 76 (15.4) | 78 (17.3) |
| Maternal BMI (kg/m2) | mean (SD) | 26.2 (5.0) | 26.6 (5.6) |
| Gravidity | median (range) | 1.8 (0 - 8) | 2.0 (0 - 6) |
| Parity | median (range) | 1.2 (0 - 6) | 1.5 (0 - 6) |
| | | | |
| Gestational age at sample collection (days) | mean (SD) | 80.9 (6.1) | 81.0 (6.1) |
| Gestational age at delivery (days) | mean (SD) | 279.8 (8.6) | 279.0 (8.1) |
| | | | |
| Birth weight (g) | mean (SD) | 3574 (433.8) | 3618 (516.1) |
| Birth weight percentile | mean (SD) | 52.47 (26.80) | 58.79 (27.77) |
| Large for gestational age (> 90 percentile) | N (%) | 9 (9.4%) | 6 (12.5%) |

### Analysis of sFPR4

The concentration of sFRP4 was determined by ELISA according to the manufacturer's instructions for use (Cloud-Clone Corp., Houston, TX). In short; a microtiter plate provided in the kit pre-coated with an antibody specific to sFRP4 is incubated with the appropriate diluted standards and serum samples. After this initial incubation and aspiration of the wells the biotin-conjugated antibody specific to sFRP4 is incubated. Next, an avidin conjugated to Horseradish Peroxidase is added and incubated after the plate was washed. After this incubation and thorough washing of the wells 3,3',5,5'-tetramethylbenzidine substrate solution is added. Those wells that contain sFRP4, the biotin-conjugated secondary antibody and HRP-conjugated avidin will exhibit a change in color. This color change has a direct correlation with the concentration sFRP4 present in the well. After addition of sulphuric acid the color change is stopped and measured spectrophotometrically at a wavelength of 450nm and 570 nm. The concentration of sFRP4 in the samples was then calculated by comparing the O.D. of the samples to that of the standard curve.

The serum samples were coded and evaluated by the investigator, who was blinded for the fact whether the sample was from an individual who developed GDM or a control.

### Statistical analysis

Statistical analysis was performed by Mann Whitney U analysis using GraphPad Prism (GraphPad Software, La Jolla, CA) and MedCalc (MedCalc Software bvba, Ostend, Belgium).

### Results

In all maternal serum samples the sFRP4 concentration was determined by ELISA. Five samples had to be excluded from evaluation because of the fact that the values were below the detection limit of the assay (62.5 pg/ml). Values from 49 GDM and 96 control samples could be included in the evaluation. This revealed a comparable median of 91.2 ng/ml vs. 93.0 ng/ml for the GDM serum samples and the serum of healthy pregnancies, respectively. Although the median was comparable, the difference in sFRP4 serum concentration between the two groups was found to be significant (P-value = 0.030, Mann Whitney test). This significance was influenced by taking the BMI of the expecting women into account. Multiplying the sFRP4 concentration with the BMI resulted in a change of the p-value to 0.0209 (Mann Whitney test). Importantly, the results show that a serum sFRP4 concentration of above 130 ng/ml at the end of the first trimester is associated with an almost four (3.75)-fold higher risk (OR) to develop GDM later in pregnancy. Where BMI alone is known to have a OR of 2.1 for overweight and 2.4 for obese women (Kim SY, et al. Am J Public Health, 2010; 100:1047-1052).

**Table 2. Statistics of the maternal serum sFRP4 concentration (GDM, N = 49) and (healthy pregnancy, N = 96) control groups at the end of the first trimester.**

| | **GDM [ng/ml]** | **Control [ng/ml]** |
|---|---|---|
| Minimum | 35,80 | 4,30 |
| 25% Percentile | 71,75 | 53,73 |
| Median | 91,20 | 93,00 |
| 75% Percentile | 175,9 | 113,9 |
| Maximum | 501,4 | 566,4 |
| | | |
| Lower 95% CI of mean | 105,7 | 87,51 |
| Upper 95% CI of mean | 154,6 | 119,7 |

Weight and especially weight-correlated BMI is known to be a risk factor to develop GDM. Because the patients were pre-selected and matched for baseline characteristics as indicated in Table 1, no significant differences in gestational age, weight and BMI were evident at the time of sampling. Both study groups were, therefore, comparable for all parameters. The predictive value of sFRP4 in relation to GDM is independent of BMI (see Table 3). The results show that a serum sFRP4 concentration of above 130 ng/ml at the end of the first trimester is associated with a3.75-fold higher risk (OR) to develop GDM later in pregnancy. BMI alone is known to have a OR of 2.1 for overweight and 2.4 for obese women (Kim SY, et al., 2010. Am J Public Health 100:1047-1052).

When a selection is made within the data on the basis of higher BMI, such as above 25 (overweight) or above 30 (obese), the predictive value of the marker is even stronger, respectively 4.1 and 5.8 (see Table 3). Predictive value, odds ratio, and sensitivity are going up, this in relation with loss in specificity.

**Table 3. Influence of selecting for higher BMI within the study population.**

| | | **Mean BMI** | | **Sensitivity** | **Specificity** | **OR** |
|---|---|---|---|---|---|---|
| | | **Unaffected** | **GDM** | | | |
| **Total group** | **sFRP4 >130** | 26.4 | 26.6 | 43% | 83% | 3.75 |
| **BMI >25** | **All** | 29.9 | 30.3 | 59% | 43% | 1.11 |
| **BMI >25** | **sFRP4 >130** | 29.9 | 30.3 | 55% | 77% | 4.10 |
| **BMI >30** | **All** | 33.2 | 33.4 | 29% | 76% | 1.25 |
| **BMI >30** | **sFRP4 >130** | 33.2 | 33.4 | 71% | 70% | 5.80 |

## Claims

1. A method of typing a pregnant female subject as being at risk of developing gestational diabetes, the method comprising:
a) determining a level of expression of Secreted Frizzled-Related Protein 4 (sFRP4) in a sample which has been obtained from the subject;
b) comparing the determined level of expression of sFRP4 with a level of expression of sFRP4 in a reference sample; and
c) typing said subject as being at risk of developing gestational diabetes if the level of expression of sFRP4 is increased in the sample of the subject, when compared to the reference sample.

2. The method according to claim 1, wherein the biological sample is a bodily fluid, preferably blood.

3. The method according to claim 1 or claim 2, wherein the biological sample is serum.

4. The method according to any one of the previous claims, wherein the biological sample is obtained before 20 weeks of gestation.

5. The method according to any one of the previous claims, wherein a level of expression of sFRP4 is determined by a biosensor, preferably by using a disposable cartridge.

6. The method according to any one of the previous claims, wherein a level of expression of sFRP4 protein is determined.

7. The method according to any one of the previous claims, wherein a level of expression of sFRP4 is determined by Enzyme-Linked Immuno Sorbent Assay (ELISA).

8. A dietary meal plan and/or physical activity for use in a method of treatment of a subject that has been typed as being at risk of developing gestational diabetes according to the methods of claims 1-7.

9. Metformin, a sulfonylurea, a thiazolidinedione, a DPP-4 inhibitor, a GLP-1 receptor agonist, and/or a SGLT2 inhibitor, for use in a method of treatment of a subject that has been typed as being at risk of developing gestational diabetes according to the methods of claims 1-7.

10. Insulin for use in a method of treatment of a subject that has been typed as being at risk of developing gestational diabetes according to the methods of claims 1-7.

11. Use of a kit for assessing a pregnant female subject for susceptibility to gestational diabetes, said kit comprising:
a device for collecting a test sample from a patient; and
reagents for measuring a level of expression of Secreted Frizzled-Related Protein 4 (sFRP4) in said sample.

12. Use of the kit according to claim 11, wherein the reagents for determining a level of expression of sFRP4 are reagents for real-time PCR or reagents for an immunochemical assay.

13. Use of the kit according to claim 11 or claim 12, wherein the reagents for determining a level of expression of sFRP4 include a receptacle that is coated with an antibody specific to sFRP4.

14. Use of the kit according to any one of claims 11-13, wherein the receptacle is a microtiter plate.

15. Use of an antibody specific to sFRP4 in an in vitro method for determining a risk of a female subject of developing gestational diabetes.

## Patentansprüche

1. Verfahren zum Einstufen einer schwangeren weiblichen Testperson als gefährdet, einen Schwangerschaftsdiabetes zu entwickeln, wobei das Verfahren Folgendes umfasst:
a) Bestimmen eines Expressionsgrads von sekretiertem frizzled-verwandtem Protein 4 ("Secreted Frizzled-Related Protein 4"; sFRP4) in einer Probe, die von der Testperson erhalten worden ist;
b) Vergleichen des bestimmten Expressionsgrads von sFRP4 mit einem Expressionsgrad von sFRP4 in einer Referenzprobe; und
c) Einstufen der Testperson als gefährdet, einen Schwangerschaftsdiabetes zu entwickeln, wenn der Expressionsgrad von sFRP4 in der Probe der Testperson im Vergleich zur Referenzprobe erhöht ist.

2. Verfahren nach Anspruch 1, wobei die biologische Probe eine Körperflüssigkeit, vorzugsweise Blut, ist.

3. Verfahren nach Anspruch 1 oder Anspruch 2, wobei die biologische Probe Serum ist.

4. Verfahren nach einem der vorhergehenden Ansprüche, wobei die biologische Probe vor 20 Schwangerschaftswochen erhalten wird.

5. Verfahren nach einem der vorhergehenden Ansprüche, wobei ein Expressionsgrad von sFRP4 durch einen Biosensor, vorzugsweise unter Verwendung einer Einwegkartusche, bestimmt wird.

6. Verfahren nach einem der vorhergehenden Ansprüche, wobei ein Expressionsgrad von sFRP4-Protein bestimmt wird.

7. Verfahren nach einem der vorhergehenden Ansprüche, wobei der Expressionsgrad von sFRP4 durch einen enzymgebundenen Immunsorbens-Assay (ELISA) bestimmt wird.

8. Diätetischer Ernährungsplan und/oder körperliche Aktivität zur Verwendung in einem Verfahren zur Behandlung einer Testperson, die nach den Verfahren der Ansprüche 1 bis 7 als gefährdet eingestuft worden ist, einen Schwangerschaftsdiabetes zu entwickeln.

9. Metformin, ein Sulfonylharnstoff, ein Thiazolidindion, ein DPP-4-Inhibitor, ein GLP-1-Rezeptoragonist und/oder ein SGLT2-Inhibitor zur Verwendung in einem Verfahren zur Behandlung einer Testperson, die nach den Verfahren der Ansprüche 1 bis 7 als gefährdet eingestuft worden ist, einen Schwangerschaftsdiabetes zu entwickeln

10. Insulin zur Verwendung in einem Verfahren zur Behandlung einer Testperson, die nach den Verfahren der Ansprüche 1 bis 7 als gefährdet eingestuft worden ist, einen Schwangerschaftsdiabetes zu entwickeln.

11. Verwendung eines Kits zur Beurteilung einer schwangeren weiblichen Testperson auf Anfälligkeit für Schwangerschaftsdiabetes, das Kit umfassend:
Vorrichtung zum Sammeln einer Testprobe von einer Patientin; und
Reagenzien zum Messen des Expressionsgrads von sekretiertem frizzled-verwandtem Protein 4 (sFRP4) in der Probe.

12. Verwendung des Kits nach Anspruch 11, wobei die Reagenzien zum Bestimmen des Expressionsgrads von sFRP4 Reagenzien für die Echtzeit-PCR oder Reagenzien für einen immunochemischen Assay sind.

13. Verwendung des Kits nach Anspruch 11 oder Anspruch 12, wobei die Reagenzien zum Bestimmen des Expressionsgrads von sFRP4 ein Gefäß umfassen, das mit einem für sFRP4 spezifischen Antikörper beschichtet ist.

14. Verwendung des Kits nach einem der Ansprüche 11 bis 13, wobei das Gefäß eine Mikrotiterplatte ist.

15. Verwendung eines Antikörpers, der für sFRP4 spezifisch ist, in einem in vitro-Verfahren zum Bestimmen des Risikos einer weiblichen Testperson für die Entwicklung eines Schwangerschaftsdiabetes.

## Revendications

1. Méthode de typage d'un sujet de type femme enceinte comme à risque de développer un diabète gestationnel, la méthode comprenant :
a) la détermination d'un niveau d'expression d'une protéine 4 sécrétée apparentée à Frizzled (sFRP4) dans un échantillon provenant du sujet ;
b) la comparaison du niveau d'expression de sFRP4 déterminé avec un niveau d'expression de sFRP4 dans un échantillon de référence ; et
c) le typage dudit sujet comme à risque de développer un diabète gestationnel si le niveau d'expression de sFRP4 est supérieur dans l'échantillon du sujet, comparativement à l'échantillon de référence.

2. Méthode selon la revendication 1, dans laquelle l'échantillon biologique est un fluide corporel, de préférence le sang.

3. Méthode selon la revendication 1 ou la revendication 2, dans laquelle l'échantillon biologique est le sérum.

4. Méthode selon l'une quelconque des revendications précédentes, dans laquelle l'échantillon biologique est obtenu avant 20 semaines de gestation.

5. Méthode selon l'une quelconque des revendications précédentes, dans laquelle un niveau d'expression de sFRP4 est déterminé par un biocapteur, de préférence à l'aide d'une cartouche jetable.

6. Méthode selon l'une quelconque des revendications précédentes, dans laquelle un niveau d'expression de protéine sFRP4 est déterminé.

7. Méthode selon l'une quelconque des revendications précédentes, dans laquelle un niveau d'expression de sFRP4 est déterminé par dosage d'immunoabsorption par enzyme liée (ELISA).

8. Programme de repas diététiques et/ou d'activité physique destiné à être utilisé dans une méthode de traitement d'un sujet qui a été typé comme à risque de développer un diabète gestationnel selon les méthodes des revendications 1 à 7.

9. Metformine, sulfonylurée, thiazolidinedione, inhibiteur de DPP-4, agoniste du récepteur GLP-1, et/ou inhibiteur de SGLT2, pour leur utilisation dans une méthode de traitement d'un sujet qui a été typé comme à risque de développer un diabète gestationnel selon les méthodes des revendications 1 à 7.

10. Insuline pour son utilisation dans une méthode de traitement d'un sujet qui a été typé comme à risque de développer un diabète gestationnel selon les méthodes des revendications 1 à 7.

11. Utilisation d'un kit pour évaluer la prédisposition d'un sujet de type femme enceinte à un diabète gestationnel, ledit kit comprenant :
un dispositif pour prélever un échantillon d'essai chez une patiente ; et
des réactifs pour mesurer un niveau d'expression de la protéine 4 sécrétée apparentée à Frizzled (sFRP4) dans ledit échantillon.

12. Utilisation du kit selon la revendication 11, dans laquelle les réactifs pour déterminer un niveau d'expression de sFRP4 sont des réactifs pour PCR en temps réel ou des réactifs pour dosage immunochimique.

13. Utilisation du kit selon la revendication 11 ou la revendication 12, dans laquelle les réactifs pour déterminer un niveau d'expression de sFRP4 comprennent un réceptacle qui est revêtu d'un anticorps anti-sFRP4.

14. Utilisation du kit selon l'une quelconque des revendications 11 à 13, dans laquelle le réceptacle est une plaque de microtitrage.

15. Utilisation d'un anticorps anti-sFR4 dans une méthode in vitro de détermination d'un risque chez une patiente de développer un diabète gestationnel.
